# EUROPEAN PATENT SPECIFICATION

(11) **EP 2 490 681 B1**
(45) Date of publication and mention of the grant of the patent: **04.11.2015**
(21) Application number: 10778746.7
(22) Date of filing: 20.10.2010
(51) Int. Cl.: A61K 31/137, A61K 9/00, A61K 31/58

(54) **THE PHARMACEUTICAL COMPOSITION IN DRY POWDER FORM FOR INHALATION**
PHARMAZEUTISCHE TROCKENPULVERZUSAMMENSETZUNG ZUM INHALIEREN
COMPOSITION PHARMACEUTIQUE SOUS FORME DE POUDRE SECHE

(30) Priority: 20.10.2009 TR 200907913
(43) Date of publication of application: 29.08.2012
(73) Proprietor: Sima Patent ve Lisanslama Hizmetleri Ltd.Sti., Esenler/Istanbul (TR)
(72) Inventor: BILGIC, Mahmut, 34220 Esenler-Istanbul (TR)
(86) International application number: PCT/TR2010/000209
(87) International publication number: WO 2011/049540

(56) References cited:
- WO-A1-01/78739
- WO-A1-2005/053648
- WO-A1-2007/012871
- WO-A2-2008/102128
- US-A1- 2003 018 019
- US-A1- 2004 009 963
- US-A1- 2009 314 291

## Description

The present invention is related to pharmaceutical compositions in dry powder form comprising β-agonists and corticosteroid drugs and the blister pack which include them and use of these for the treatment of the respiratory diseases.

These molecules named as generally β₂-agonists which are categorized into two grups such as long-acting and short-acting leads to opening of bronchs by causing relaxation of smooth muscles around air vessels. With the help of this property, these drugs are indicated for use in treatment of asthma and chronic obstructive pulmonary disorder (COPD).

These drugs indicate effects in a very short time, for instance a few minutes after their inhalation and their effects can continue for 4 hours. They should be often taken due to their short-lasting remedial effects.

The drugs included in this group are categorized into two main groups as mentioned before. The first of them is β₂-agonists comprising salbutamol, levosalbutamol, prosaterol, fenoterol, terbutaline, pirbuterol, metoproterenol, bitolterol mesilate which affects rapidly but lose its effect in a short time. The second group consists of salmeterol, formoterol, bambuterol and clenbuterol and they affect less slowly compared to the other group and are characterized by their long-lasting effects.

The drugs named as Albuterol (Ventolin, Proventil), metaproterenol (Alupent), pirbuterol (Maxair), terbutaline (Brethaire), isoetharine (Bronkosol), and Levalbuterol (Xopenex), salmeterol xinafoate (Serevent) in the markets can be examples of the pharmaceutical compositions comprising the molecules included in this group.

In 2005, US Food and Drug Administration (FDA) claimed that a lot of long-acting β₂-agonists drugs includes growling symptom in the patients. Moreover, in a study carried out by Cornell and Stanford Universities, it has been found that usage for a long time and/or taking β-agonists in a high dosage in the COPD treatment increases health problems resulting from respiratory tract. As it is seen from this, different methods should be applied for the use of β₂-agonists in the treatment of asthma and COPD.

Moreover, another drug group that is often used for the treatment of asthma and COPD is corticosteroids. The molecules included in this group used since approximately 1950s makes the air vessels open by decreasing mucus and this results in controlling inflammation that forms the basis of the asthma and makes the problems of asthma patients disappear as a result. When the drugs included in this group are taken systemically, they cause serious side effects such as osteoporosis, high cholesterol, oedema, headache, sleep problems, some skin problems and growth retardation in children. Therefore, delivery methods in which low amount of it enters to systemic circulation as far as possible should be preferred. In asthma treatment, delivery of these kinds of molecules is applied by inhalation route in order to decrease the side-effect and to transport the maximum dose to the lung as much as possible.

As it is mentioned above, β-agonists drugs trigger respiratory diseases, for preventing the side effects of these substances that should be used in the asthma treatment, the combination of them with the other substances affecting on the same disease is beneficial for both decreasing the amount of β₂-agonists to be taken and the benefits of the synergic effects of the combination of two different drugs for the patients. For that reason, it is found that using β₂-agonists and corticosteroids together provides both decrease in the amount of β₂-agonists and opening bronchus with the positive effect of this drug, and preventing the inflammation resulting from asthma by using corticosteroid.

Ideally, while the dosage forms of the drugs indicating the remedial effects in the body are designed, delivery methods providing that the drug affects directly and rapidly and accordingly indicates the desired affects in lower dosages and thus indicates side effects less are preferred. Especially, considering the serious side effects of corticosteroids in the systemic intake, it is seen that it is very important to transmit this combination which is thought to be transmitted by respiratory route effectively. In the prior art, one of the problems mostly emphasized is these delivery methods and improving the devices.

Generally, there are three different methods and inhalation devices which are suitable for use in the drug delivery by inhalation route. These are; (a) nebulization devices, (b) inhalators including pressurized gas and (c) dry powder inhalators.

The nebulization devices are not often preferred due to their large sizes and due to the fact that they sometimes cause infection. The inhalators including pressurized gas which is the other method are more advantageous than nebulizers via being easily moveable, but the largest part of the dose accumulates in different points so as to join the systemic circulation before it can not reach the lung in these devices and only 10-20% of the drug taken reaches to the target area in the best situations, in other words, in the case of taking the drug by 100% efficiency of the patient. An important factor causing this situation is that the carrier gas used in these devices have cold freon effect and as a consequence the respiration of the patient stops for a short period while using the drug and the drug accumulates in the mouth instead of reaching to the target area by breathing of the drug. Considering the side effect of the corticosteroid that is mentioned before, it is seen that using the inhalators including pressurized gas in delivery of these group drugs is not a true choice for the patients.

The inhalators including dry powder come into prominence via their usage easiness. These devices both can be moved to everywhere with the help of having small size and do not cause the cold freon effect due to not having carrier gas. Moreover, contrary to the inhalators including pressurized gas, it not required that pressing and breathing processes are at the same time and it plays an important role on taking the drug of the patients effectively.

It is possible that the inhalators comprising dry powder are categorized into three groups, one of them is the inhalators comprising disposable capsule. These are not mostly preferred due to the fact that a new capsule should be placed before each use. Another inhalator including dry powder is the inhalators having reservoir comprising dry powder. These are beneficial via providing numerously dosing but these devices are not sufficient for the uniformity of the dosage. Another type of inhalators comprising dry powder is the devices in which drug is placed in the blister. The devices including blister; (a) are more advantageous than the devices comprising reservoir since it provides intake of the right dose amounts via filling each dose into different blister cavities in the factory.

Moreover, different formulations which can remain stable, are still needed to be improved for transporting the effective usage dose to the lung.

The inventors have faced with the frequent problems about not being able to provide the amount of effective dose in the inhalation formulations. For solving this problem, several methods and devices were tried.

In the prior art, the effective delivery of the dry powder formulations used in the treatment of respiratory diseases to the lungs is one of the problems emphasized mostly. Each blister in said blister strip package is pierced with a piercing device or it is opened by peeling the lid sheet and the dry powder formulation become ready for being withdrawn to the lung.

The dry powder inhaler comprising blisters that are opened by piercing or peeling requires additional mechanic components and this situation makes usage of the device difficult for the patient by increasing the size, volume and complexity of the dry powder inhaler. Moreover, in the blisters opened by tearing, the amount of the dry powder formulation remained without inhaling in the cavity due to the roughness formed resulting from tearing increases and hence the rate of utilization from the present dry powder formulation decreases.

Moreover, in order to obtain maximum benefit from the dry powder formulation that will be administered to the lungs via inhalation, the blister cavity wherein the dry powder is stored must have the maximum discharge capacity.

In prior art, several methods are tried for delivery of combinations which comprises two different active agents for example; to provide the multi-active combination which is defined in WO2007012871A, WO2007068900A, WO20050114089A numbered patents, there are dry powder inhalers in which two active agents are contained in two separate blister strips.

In these devices, it is provided that two blister strips, each of which containing a different active agent, are opened before inhalation and active agents are mixed in manifold part of the device before the delivery of the drugs to lungs of the patient.

However, since two blisters will be used, after the inhalation there will be uninhaled active agent remained in each of the blister and this way it will not be possible to deliver required dose of dry powder formulations

The inventors have suprisingly found that the simultaneos use of dry powder compositions comprising β₂-agonists and corticosteroid drugs which are contained in a single aliminum blister pack in a multiple dose inhaler for dry powder form provides the effective and certain dose of the active agents and it causes the lowest side effects and maximum therapeutic benefit of the active agents as a result of synergistic effect and efficiency on delivery of the drugs.

The present invention discloses a pharmaceutical composition in dry powder form containing β₂-agonists and corticosteroid drugs which are stored in a peelable blister strip together and storage of this pharmaceutical composition in a peelable aliminum blister strip pack and administration of this composition to the patient by the dry powder inhaler device.

For the purpose of providing multiple dosing and thus providing usage easiness to the patients, the use of blister strip package which is trustable in terms of hygiene and also removing the possibility of dry powder moistening and additionally helps the dosage to be adjusted has been preferred.

The blister strip package which is preferred to be used in the present invention, consists of two layers which are peeled away from each other. At the base sheet, the blister cavities comprising the dry powder formulations are present. The lid sheet acts as a lid and is peeled apart from the base sheet to open a blisters in order to make the dry powder formulation get ready for inhalation.

According to the present invention, it is found that the most convenient method for supplying the effective dose to target area is that the pharmaceutical composition being in the form of dry powder and stored in a peelable blister strip.

According to the invention, it is seen that when the pharmaceutical composition comprising effective amount of at least one β₂-agonist and at least one corticosteroid drugs is administered from a peelable blister pack, the negative affects resulting in adhesive force of the fine micronized dry powder is minimized and the delivery rate of drug composition to lung is increased by comparison with the dry powder delivery methods in which the drug composition is stored in a capsule or reservoir or two active agents are stored in two blisters which are apart from each other.

According to an aspect of the present invention provides a method comprising that the pharmaceutical composition including effective amounts of the combination of β₂-agonist and and corticosteroid drugs and optionally a pharmaceutically acceptable carrier is stored in blister strip package, is delivered by a dry powder inhalation device from this blister strip for the treatment of the patients having respiratory diseases, especially allergic diseases.

According to another aspect of the present invention, a pharmaceutical drug composition in a dry powder form comprising the combination of β₂-agonist and corticosteroid is inhaled effectively.

According to another aspect of the present invention, a combination of β₂-agonist and corticosteroid in the same dry powder formulation which can be inhaled with a maximum discharge capacity is provided.

According to another aspect of the present invention, a dry powder formulation containing the combination of a β2-agonists and corticosteroid, which is administered by a dry powder inhaler from a peelable blister strip for achieving more affective inhalation, is provided.

According to another aspect of the invention, a dry powder drug comprising the combination of β₂-agonist and corticosteroid which is inhaled simultaneously from a single blister to achieve the effective dose range is provided.

According to another aspect of the present invention, it is provided that the inhalation of a dry powder formulation containing the combination of a β2-agonist and corticosteroid is achieved by a multiple dose dry powder inhaler which is simple, manufactured inexpensively and used safely.

The dry powder drug containing the combination of β₂-agonist and corticosteroid can optionally include one or more carriers.

According to the invention, the carrier in the dry powder drug can be selected from a group comprising monosaccharides, disaccharides, polysaccharides and oligosaccharides. Preferably lactose is used.

According to the invention the carrier is present in the range of 0-50 mg in the dry powder drug.

The cavity volume of the blister comprising the combination of β₂-agonists and corticosteroid combination in dry powder form and mentioned lactose content, is in the range of 20 to 30 mm³, preferably of 21 to 25 mm³, most preferably of 22 to 23 mm³

According to the invention, the blister strip which consists of blisters which have a cavity volume in the range of 20 to 30 mm³ preferably in the range of 21 to 25 mm³, most preferably in the range of 22-23 mm³, is used for providing efficient inhalation by dealing with the drawbacks defined herein before. Additionally, each blister cavity having the volume described above is filled up to 25-100 %, preferably up to 70-100 %, most preferably up to 90-100 % of the said volume.

Lid sheet and base sheet of said blister strip are closed very tightly to provide impermeability by using any suitable method.

The lid and base sheet comprising the blister strip in accordance with the present invention consists of several layers. Polymeric layers, aluminium foil and preferably Aclar® fluoropoylmer film; are among the layers that form the lid and base sheet.

Aclar® fluoropolymer film is a polymeric film which used in a blister strip and provides excellent moisture barrier. This chemically inert polymeric film does not cause any change in taste of formulation when it is in contact with dry powder formulation. It can easily form a layered structure with various other polymeric layers. It has a structure which is appropriate to the treatment of heat.

Desiccant agents are optionally added to the polymeric layers in order to reduce moisture and gas permeability of the polymeric layers for protection of stability of the dry powder formulation contained in the blisters which are arranged adjacently. Some examples of the desiccant agents are silica gel, zeolite, alumina, baucsite, anhydrous calcium sulfate, activated carbon, clay capable of absorbing water.

Aluminium foil is used in both lid sheet and base sheet of the blister strip to provide high humidity and gas protection because of that aluminium foil is conventionally used in both lid sheet and base sheet of blister strip package with high humidity and gas protection. These layers must have the sufficient thickness which provides the protection for the stability of humidity sensitive dry powder formulation which is stored in blister cavity. Because of this reason, the thickness of aluminium foil that is used in lid sheet and base sheet of the blister strip package is in the range of 10 to 40 µm, preferably of 15 to 40 µm.

The polymeric layers contained by lid sheet and base sheet of the blister strip according to the present invention are the layers which are made from same or different polymers. The thickness of these polymeric layers depends on the type of polymeric substance used and its properties. Therefore, the thickness of each polymeric layer which is used in lid sheet and base sheet of said blister strip is in the range of 15 to 60 µm, preferably of 20 to 35 µm depending on the type of used polymer.

When the inside layer of the cavity is made up of aluminium foil a part of the dry powder formulation remains in the cavity uninhaled due to electrostatic forces, therefore the layer covering the inside of the cavity is the polymeric layer to prevent the formation of this adhesive force.

According to the present invention, the polymers used for forming polymeric layers are preferably selected from a group comprising thermo-plastic polymers such as polyethylene, polypropylene, polystyrene, polyolefin, polyamide, polyvinyl chloride, polyurethane or synthetic polymers.

Morever, blisters which form the blister pack may be in any shape by the purpose of providing the above-mentioned features.

Corticosteroid drugs which are one of the group of active agents of the dry powder formulation according to the invention can be selected from a group comprising, bot not limited with,triamcinolone, beclomethazone, mometasone, ciclesonid, budesonide and flutikasone or their pharmaceutically acceptable salts, solvates, derivatives.

β₂-agonists which are one of the group of active agents of the dry powder formulation according to the invention can be selected from a group comprising, but not limited with, salbutamol, levosalbutamol, prosaterol, fenoterol, terbutaline, pirbuterol, metoproterenol, bitolterol mesilate, salmeterol, formoterol, bambuterol and clenbuterol, arformeterol ve Isoetarin and their pharmaceutically acceptable salts, esters.

The dry powder formulation in blister cavities of said peelable blister strip is produced according to the prior art. The particle size of the active agents in dry powder formulation is less than 20µm. Lactose is used as a pharmaceutical excipient. The different sized pharmaceutical excipient particles comprising fine or more coarse particles which are various particle size distribution, can be used to provide effective dry powder inhalation.

The weight ratio of corticosteroid or a pharmaceutically acceptable salt thereof and β₂-agonists or a pharmaceutically acceptable salt thereof in the dry powder formulation of the invention is in the range of 1:1 and 100: 1, preferably 1:1 and 60:1.

The pharmaceutical composition in dry powder form in each blister of said peelable blister strip contain 1-700µg of corticosteroid or a pharmaceutically acceptable salt thereof, 1-50µg of β₂-agonist or a pharmaceutically acceptable salt thereof.

Pharmaceutical composition of corticosteroid and β₂-agonist combination in accordance with present invention can be used for the treatment of many respiratory diseases especially asthma, chronic obstructive pulmonary disorder (COPD) and allergic rhinitis. Accordingly, the respiratory diseases include but are not restricted to; allergic or non-allergic asthma in various phases, acute lung injury (ALI), acute respiratory distress syndrome (ARDS), exacerbation of airways hyperactivity, bronchiectasis, chronic obstructive pulmonary, airways or lung diseases (COPD, COAD or COLD) including emphysema and chronic bronchitis, pneumoconiosis, aluminosis, anthracosis, asbestosis, chalicosis, ptilosis, siderosis, silicosis, tabacosis and byssinosis. The treatment of said diseases may be prophylactic or symptomatic. In addition to this, the pharmaceutical composition in accordance with the present invention is used especially for the symptomatic treatment of asthma, allergic rhinitis and COPD.

## Claims

1. A pharmaceutical composition containing a comibination comprised of a corticosteroid and a β₂-agonist **characterized in that**
• the components of said combination are in the dry powder form,
• the average particle size of the active agents is less than 20 µm,
• two active agents contained in said pharmaceutical composition are together with lactose as carrier and they are stored in a peelable, aluminium blister strip, wherein the cavity volume of the blister is in the range of 22 to 23 mm3 and each blister cavity is filled up to 70-100 % of said volume,
• said pharmaceutical composition is suitable to be administered to a patient by a device suitable for dry powder.

2. A pharmaceutical composition according to claim 1, wherein corticosteroid used can be chosen from a group comprising, but not limited with, triamcinolone, beclomethazone, mometasone, ciclesonid, budesonide and fluticasone or their pharmaceutically acceptable salts, solvates, derivatives.

3. A pharmaceutical composition according to claim 1, wherein β₂-agonists used can be chosen from a group comprising, but not limited with, salbutamol, levosalbutamol, prosaterol, fenoterol, terbutaline, pirbuterol, metoproterenol, bitolterol mesilate, salmeterol, formoterol, bambuterol and clenbuterol and their pharmaceutically acceptable salts, esters, solvates.

4. A pharmaceutical composition in the dry powder form according to claim 1 wherein said carrier is in an amount of 0 to 50 mg.

5. A pharmaceutical composition in the dry powder form according to any one of the previous claims, the weight ratio of a corticosteroid or a pharmaceutically acceptable salt thereof to a β₂-agonists or a pharmaceutically acceptable salt thereof is in the range of 1:1 to 100: 1, preferably 1:1 to 60:1.

6. A pharmaceutical composition in the dry powder form according to one of the previous claims wherein each blister of the blister strip contains 1-700µg of corticosteroid or a pharmaceutically acceptable salt thereof, and 1-50µg of β₂-agonist or a pharmaceutically acceptable salt thereof.

7. A pharmaceutical composition according to claim 1 is used for the symptomatic and pyrophylactic treatment of chronic obstructive pulmonary disease (COPD), allergic rhinitis, allergic or non-allergic asthma in various phases, acute lung injury (ALI), exacerbation of airways hyperactivity, bronchiectasis, emphysema, chronic obstructive pulmonary, airways or lung diseases (COPD, COAD or COLD) including emphysema and chronic bronchitis, pneumoconiosis, aluminosis, anthracosis, asbestosis, chalicosis, ptilosis, siderosis, silicosis, tabacosis and byssinosis.

8. A pharmaceutical composition according to claim 7 is preferably used for the symptomatic treatment of allergic asthma and COPD

## Patentansprüche

1. Eine pharmazeutische Zusammensetzung, die eine Kombination aus einem Kortikosteroid und einem β2-Agonisten umfasst und **dadurch gekennzeichnet, dass**
• die Komponenten der genannte Kombination in der Form eines trockenen Pulvers sind,
• die durchschnittliche Teilchengröße der Wirkstoffe weniger als 20 µm ist,
• zwei Wirkstoffe, die in der genannten pharmazeutischen Zusammensetzung enthalten sind, zusammen mit Laktose als Trägerstoff sind, und in einem abziehbaren, Aluminium-Blisterstreifen gelagert werden, wobei das Hohlraumvolumen des Blisters im Bereich von 22 bis 23 mm3 ist und jeder Hohlraum im Blister bis zu %70-100 des genannten Volumens aufgefüllt wird.
• die genannte pharmazeutische Zusammensetzung geeignet ist, um einem Patienten durch ein für Trockenpulver geeignetes Gerät verabreicht zu werden.

2. Eine pharmazeutische Zusammensetzung nach Anspruch 1, wobei das verwendete Kortikosteroid aus einer Gruppe einschließlich, aber nicht beschränkt auf Triamcinolon, Beclometason, Mometason, Ciclesonid, Budesonid und Fluticasone oder ihre pharmazeutisch annehmbare Salze, Solvate, Derivate ausgewählt werden kann.

3. Eine pharmazeutische Zusammensetzung nach Anspruch 1, wobei die verwendeten β2-Agonisten aus einer Gruppe einschließlich, aber nicht beschränkt auf Salbutamol, Levosalbutamol, Prosaterol, Fenoterol, Terbutalin, Pirbuterol, Metoproterenol, Bitolterol-Mesilat, Salmeterol, Formoterol, Bambuterol und Clenbuterol und ihre pharmazeutisch annehmbare Salze, Ester, Solvate ausgewählt werden könnten.

4. Eine pharmazeutische Zusammensetzung in der Form eines trockenen Pulvers nach Anspruch 1, wobei der genannte Träger in einer Menge von 0 bis 50 mg ist.

5. Eine pharmazeutische Zusammensetzung in der Form eines trockenen Pulvers nach einem der vorhergehenden Ansprüche, wobei das Gewichtsverhältnis eines Kortikosteroids oder eines pharmazeutisch annehmbaren Salzes davon an einen β2-Agonisten oder ein pharmazeutisch annehmbares Salz davon im Bereich von 1:1 bis 100:1, vorzugsweise 1:1 bis 60:1 ist.

6. Eine pharmazeutische Zusammensetzung in der Form eines trockenen Pulvers nach einem der vorhergehenden Ansprüche, wobei jeder Blister des Blisterstreifens 1-700µg von Kortikosteroiden oder ein pharmazeutisch annehmbares Salz davon und 1-50µg des β2-Agonisten oder ein pharmazeutisch annehmbares Salz davon beinhaltet.

7. Eine pharmazeutische Zusammensetzung nach Anspruch 1, wobei es zur symptomatischen und prophylaktischen Behandlung von chronisch-obstruktiver Lungenerkrankung (COPD), allergischer Rhinitis, allergischern oder nicht-allergischem Asthma in verschiedenen Phasen, akuter Lungeninsuffizienz (ALI), Exazorbation einer Aternwegs-Hyperaktivität, Bronchiektasien, Emphysem, chronischer Bronchitis, Staublunge, Aluminose, Kohlenstaublunge, Asbestose, Chalikose, Ptilose, Siderose, Silikose, Tabakstaublunge und Byssinose verwendet wird.

8. Eine pharmazeutische Zusammensetzung nach Anspruch 7, wobei es vorzugsweise für die symptomatische Behandlung von allergischem Asthma und COPD verwendet wird.

## Revendications

1. Une composition pharmaceutique comprenant une combinaison composée d'un corticostéroïde et d'un β₂-agoniste **caractérisé en ce que**
• les composants de ladite combinaison sont sous la forme de poudre sèche,
• la taille moyenne des particules des principes actifs sont moins que 20 µm,
• deux agents actifs contenus dans ladite composition pharmaceutique sont ensemble avec lactose comme le support et ils sont stockés dans une bande de blister d'aluminium, pelable; dans laquelle le volume de la cavité du blister est dans la plage de 22 à 23 mm³ et chaque cavité du blister est remplie jusqu'à 70-100 % dudit volume,
• ladite composition pharmaceutique est appropriée pour être administrée à un patient par un dispositif approprié pour la poudre sèche.

2. Une composition pharmaceutique selon la revendication 1, dans laquelle le corticostéroïde utilisé peut être choisi dans un groupe comprenant, mais non limité à, la triamcinolone, le béclométasone, la mométasone, le ciclésonide, le budésonide et la fluticasone ou leurs sels, solvates, dérivés pharmaceutiquement acceptables.

3. Une composition pharmaceutique selon la revendication 1, dans lequel les β2-agonistes utilisés peuvent être choisis dans un groupe comprenant, mais non limité à, le salbutamol, le lévosalbutamol, le prosaterol, le fénotérol, la terbutaline, le pirbutérol, le metoproterenol, le mésylate de bitoltérol, le salmétérol, le formotérol, le bambutérol et le clenbutérol et leurs sels, esters, solvates pharmaceutiquement acceptables.

4. Une composition pharmaceutique sous la forme de poudre sèche selon la revendication 1, dans laquelle ledit support est dans un montant de 0 à 50 mg.

5. Une composition pharmaceutique sous la forme de poudre sèche selon l'une quelconque des revendications précédentes, le rapport en poids d'un corticostéroïde ou un sel pharmaceutiquement acceptable de celui-ci à un β2-agoniste ou un sel pharmaceutiquement acceptable de celui-ci est dans 1a plage de 1:1 à 100:1, de préférence de 1:1 à 60:1.

6. Une composition pharmaceutique sous la forme de poudre sèche selon l'une des revendications précédentes, dans laquelle chaque blister de la bande de blister contient 1-700 µg du corticostéroïde ou d'un sel pharmaceutiquemcnt acceptable de celui-ci, et 1-50 µg du β2-agoniste ou d'un sel pharmaceutiquement acceptable de celui-ci.

7. Une composition pharmaceutique selon la revendication 1 est utilisée pour le traitement symptomatique et prophylactique de la broncho-pneunopathie chronique obstructive (BPCO), la rhinite allergique, l'asthme allergique ou non allergique dans diverses phases, le syndrome de détresse respiratoire aiguë (SDRA), l'exacerbation des voies aériennes hyperactivité, la bronchectasie, l'emphysème, la bronchite chronique, la pneumoconiose, l'aluminosis, l'anthracose, l'asbestose, le chalicosis, le ptilose, la sidérose, la silicose, la tabacosis, et la byssinose.

8. Une composition pharmaceutique selon la revendication 7 est de préférence utilisée pour le traitement symptomatique de l'asthme allergique et le BPCO.
